(19) Europäisches Patentamt
European Patent Office
Office européen des brevets

(11) Veröffentlichungsnummer : **0 106 243**
**B1**

(12) # EUROPÄISCHE PATENTSCHRIFT

(45) Veröffentlichungstag der Patentschrift : **28.05.86**

(21) Anmeldenummer : **83109794.4**

(22) Anmeldetag : **30.09.83**

(51) Int. Cl.⁴ : **C 07 D249/08**, C 07 D233/61, C 07 D231/12, C 07 D275/04, C 07 D403/06, A 01 N 43/50, A 01 N 43/653, A 01 N 43/80, C 09 D 5/14, C 14 C 9/00, B 27 K 3/34

(54) **Azolyl-methylamine, ihre Herstellung und Verwendung in mikrobiziden Mitteln.**

(30) Priorität : **13.10.82 DE 3238006**

(43) Veröffentlichungstag der Anmeldung : **25.04.84 Patentblatt 84/17**

(45) Bekanntmachung des Hinweises auf die Patenterteilung : **28.05.86 Patentblatt 86/22**

(84) Benannte Vertragsstaaten : **BE DE FR GB IT SE**

(56) Entgegenhaltungen :
**AT-B- 357 153**
**CH-A- 631 172**
**DE-B- 1 004 617**

(73) Patentinhaber : **BAYER AG**
**Konzernverwaltung RP Patentabteilung**
**D-5090 Leverkusen 1 Bayerwerk (DE)**

(72) Erfinder : **Oeckl, Siegfried, Dr.**
**Auf der Höhe 74**
**D-5060 Bergisch-Gladbach 1 (DE)**
Erfinder : **Schmitt, Hans-Georg, Dr.**
**Gustav-Freytag-Strasse 2**
**D-5090 Leverkusen 1 (DE)**
Erfinder : **Paulus, Wilfried, Dr.**
**Deswatinesstrasse 90**
**D-4150 Krefeld 1 (DE)**
Erfinder : **Genth, Hermann, Dr.**
**Am Heckerhof 60**
**D-4150 Krefeld 1 (DE)**

**EP 0 106 243 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Azolyl-methylamine, ein Verfahren zu ihrer Herstellung und ihre Verwendung in mikrobiziden Mitteln.

Aus der schweizerischen Patentschrift 631 172 ist ein Verfahren zur Herstellung von neuen 1,2-Benzisothiazolinonen-3 bekannt. Die dort hergestellten neuen Verbindungen sind beispielsweise als Arzneimittel verwendbare Substanzen.

Es wurden neue Azolyl-methylamine gefunden der Formel

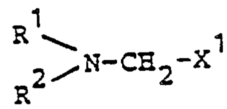

(I)

in der $X^1$ einen der Reste

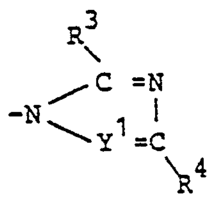 oder 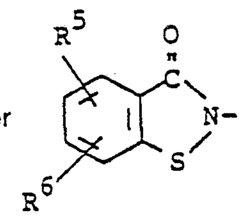

bedeutet, in denen

$Y^1$ für N oder die Gruppe

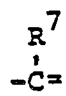

steht und

$R^3$ bis $R^7$ Wasserstoff, Halogen, Nitro, Alkyl ($C_1$-$C_{24}$), Cycloalkyl ($C_3$-$C_{12}$), Alkoxy ($C_1$-$C_{24}$), Cyano oder Aralkyl ($C_7$-$C_{24}$) bedeuten,

$R^1$ Alkyl ($C_1$-$C_{24}$), Alkenyl ($C_2$-$C_{24}$), Alkinyl ($C_2$-$C_{24}$), Cycloalkyl ($C_3$-$C_{12}$), Aralkyl ($C_7$-$C_{24}$), Aminoalkyl ($C_1$-$C_{24}$), Alkylenaminoalkylen ($C_2$-$C_{24}$), Aryl ($C_6$-$C_{18}$), Alkylaryl ($C_7$-$C_{24}$), Halogenaryl ($C_6$-$C_{18}$), Alkoxy ($C_1$-$C_{24}$), Arylalkoxy ($C_7$-$C_{24}$) oder die Gruppe

$$—CH_2X^1$$

in der $X^1$ die obengenannte Bedeutung hat, bedeutet, $R^2$ Wasserstoff oder $R^1$ bedeutet, wobei $R^1$ die obengenannte Bedeutung hat,

und wobei die Reste $R^1$ und $R^2$ in einem heterocyclischen 5- oder 6-gliedrigen Ring mit Stickstoff, Sauerstoff und/oder Schwefel als Heteroatom verknüpft sein können.

Weiterhin wurden gefunden Azolyl-methylamine der Formel

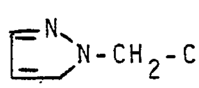 ,

in der

C für 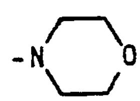 , 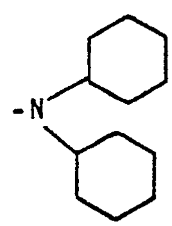 , 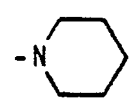 ,

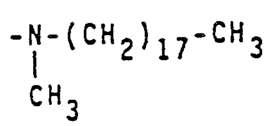 , $-N(CH_3)_2$ , $-N(C_2H_5)_2$ ;

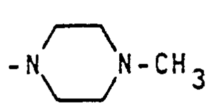 , 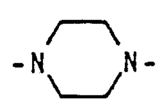 , 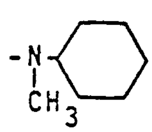 ,

(Fortsetzung)

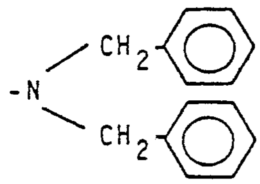, 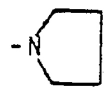, 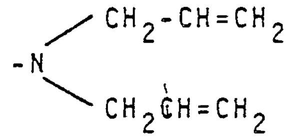,

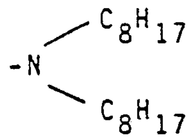, 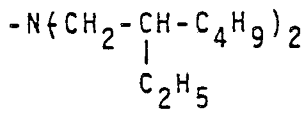,

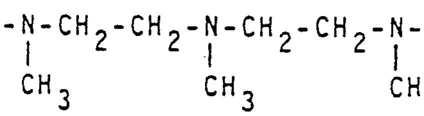 und 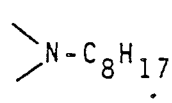 steht.

Azoyl-methylamine der Formel

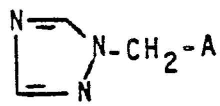,

in der

A für 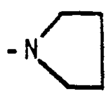; 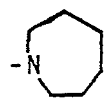 und 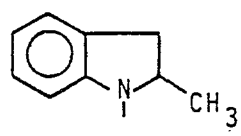 steht,

Azoyl-methylamine der Formel

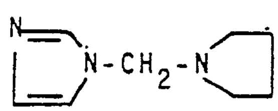 und 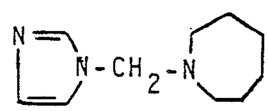 sowie

Azoyl-methylamine der Formel

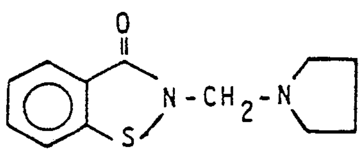 und 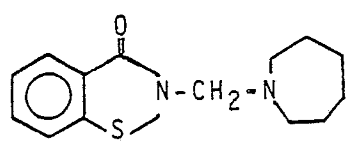.

Halogen kann hierbei erfindungsgemäß Fluor, Chlor, Brom oder Jod, bevorzugt Fluor und Chlor, bedeuten.

Alkyl kann erfindungsgemäß einen geradkettigen oder verzweigten Kohlenwasserstoffrest mit 1 bis 24, bevorzugt 1 bis 18, Kohlenstoffatomen bedeuten. Beispielsweise seien die folgenden Alkylreste genannt : Methyl, Ethyl, Propyl, Isopropyl, Butyl, tert.-Butyl, Isoamyl, 2-Ethylhexyl, Octyl, Dodecyl und Stearyl.

Alkenyl kann erfindungsgemäß ein geradkettiger oder verzweigter ungesättigter Kohlenwasserstoffrest mit 2 bis 24, bevorzugt 2 bis 18, Kohlenstoffatome bedeuten. Im allgemeinen enthalten die Alkenylreste eine oder zwei Doppelbindungen. Beispielsweise seien die folgenden Alkenylreste genannt : Propenyl, Butenyl, Hexenyl, Octenyl, Decenyl, Dodecenyl.

Alkinyl kann erfindungsgemäß einen geradkettigen oder verzweigten 3-fach ungesättigten Kohlenwasserstoffrest mit 2 bis 24, bevorzugt 2 bis 18, Kohlenstoffatomen bedeuten. Die Alkinylreste enthalten im allgemeinen eine oder zwei Dreifachbindungen. Beispielsweise seien die folgenden Alkinylreste genannt : Propinyl, Butinyl, Hexinyl, Octinyl, Dodecinyl.

Cycloalkyl kann erfindungsgemäß ein cyclischer Kohlenwasserstoffrest mit 3 bis 12, bevorzugt 5 bis 7, Kohlenstoffatomen bedeuten. Beispielsweise seien die folgenden Cycloalkylreste genannt : Cyclopentyl, Cyclohexyl, Cycloheptyl, Methylcyclohexyl.

Alkoxy kann erfindungsgemäß einen über Sauerstoff gebundenen geradkettigen oder verzweigten Kohlenwasserstoffrest mit 1 bis 24, bevorzugt 1 bis 18, Kohlenstoffatomen bedeuten. Beispielsweise seien die folgenden Alkoxyreste genannt: Methoxy, Ethoxy, Propoxy, Isopropoxy, Oktyloxy, Dodecyloxy, Polyoxyethylenoxy, Aminoethyloxy, Dimethylaminoethyloxy.

Halogenalkyl kann erfindungsgemäß einen durch Halogen substituierten geradkettigen oder verzweigten Kohlenwasserstoffrest mit 1 bis 24, bevorzugt 1 bis 18, Kohlenstoffatomen bedeuten. Halogen kann hierbei Fluor, Chlor, Brom oder Jod, bevorzugt Fluor oder Chlor, sein. Im allgemeinen können die Alkylreste durch 1 oder mehrere Halogenatome substituiert sein. Es ist aber auch möglich, daß die Alkylreste perhalogeniert sind. Beispielsweise seien die folgenden Halogenalkylreste genannt: Chlorethyl, Bromethyl, Dichlorethyl, Trichlorethyl, Chlorbutyl, Chloroctyl, Chlordodecyl.

Aralkyl kann erfindungsgemäß ein geradkettiger oder verzweigter Kohlenwasserstoffrest sein, der durch Aryl, vorzugsweise Phenyl, Naphthyl und Biphenyl, substituiert ist und der insgesamt aus 7 bis 24, bevorzugt 7 bis 12, Kohlenstoffatomen besteht. Im allgemeinen sind die Alkylreste durch 1 bis 2 Arylreste substituiert.

Aminoalkyl kann erfindungsgemäß ein durch Aminogruppen substituierter geradkettiger oder verzweigter Kohlenwasserstoffrest mit 1 bis 24, bevorzugt 1 bis 18, Kohlenstoffatomen bedeuten. Im allgemeinen sind die Aminoalkylreste durch 1 bis 10 Aminogruppen substituiert. Beispielsweise seien die folgenden Aminoalkylreste genannt: Dimethylaminomethyl, Diethylaminomethyl, Dioctylaminomethyl, Azolylaminomethyl, Dimethylaminoethyl, Dioctylaminoethyl, Aminohexyl.

Alkylenaminoalkylen kann erfindungsgemäß ein Polyamin sein, bei dem vorzugsweise 2 bis 100 Niederalkylengruppen (2 bis 12 Kohlenstoffatome) über Aminogruppen der Formel

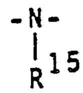

in denen $R^{15}$ Wasserstoff oder Niederalkyl bedeutet,
miteinander verbunden sind. Beispielsweise seien die folgenden Alkylenaminoalkylenreste genannt: Ethylenaminoethylen, Diethylendiaminoethylen, Triethylentriaminoethylen.

Aryl kann erfindungsgemäß ein aromatischer Rest mit 6 bis 18, vorzugsweise 6 bis 12, Kohlenstoffatomen sein. Beispielsweise seien die folgenden Arylreste genannt: Phenyl, Naphthyl und Biphenyl.

Alkylaryl kann erfindungsgemäß ein durch Alkylgruppen substituierter Arylrest mit insgesamt 7 bis 24, bevorzugt 7 bis 12, Kohlenstoffatomen sein. Im allgemeinen sind die Arylreste, bevorzugt Phenyl, Naphthyl und Biphenyl, durch 1 bis 3 Niederalkylreste (1 bis etwa 8 Kohlenstoffatome) substituiert. Beispielsweise seien die folgenden Alkylarylreste genannt: Benzyl, Phenethyl, Phenyloctyl.

Halogenaryl kann erfindungsgemäß ein durch Halogen substituierter Arylrest mit 6 bis 18, bevorzugt 6 bis 12, Kohlenstoffatomen sein. Als Halogen sei Fluor, Chlor, Brom und Jod, bevorzugt Fluor und Chlor, genannt. Im allgemeinen sind die Arylreste durch 1 bis 3 Halogenatome substituiert. Es ist aber auch möglich, daß die Arylreste perhalogeniert sind. Beispielsweise seien die folgenden Halogenarylreste genannt: Chlorphenyl, Bromphenyl, Jodphenyl, Dichlorphenyl, Trichlorphenyl, Pentachlorphenyl, Chlornaphthyl.

Arylalkoxy kann erfindungsgemäß ein durch Aryl substituierter geradkettiger oder verzweigter über Sauerstoff gebundener Kohlenwasserstoffrest mit insgesamt 7 bis 24, bevorzugt 7 bis 12, Kohlenstoffatomen sein. Im allgemeinen ist hier der Alkoxyrest durch einen bis 3 Arylreste substituiert. Beispielsweise seien die folgenden Arylalkoxyreste genannt: Benzyloxy, Phenethoxy, Phenyloctyloxy.

Es ist möglich, daß die Reste $R^1$ und $R^2$ zu einem heterocyclischen 5- oder 6-gliedrigen Ring mit Stickstoff, Sauerstoff und/oder Schwefel im Ring verknüpft sind. Im allgemeinen enthält der Ring 1 bis 3, bevorzugt 1 bis 2, Heteroatome. Beispielsweise seien die folgenden heterocyclischen Reste genannt: Piperazinyl, Methylpiperazinyl, Morpholinyl, Thiomorpholinyl, Pyrrolidinyl, Piperidinyl, Hexamethyleniminyl, Indolinyl, 2-Methyl-indolinyl, Isoindolinyl.

Erfindungsgemäß werden Azolylmethylamine der Formel

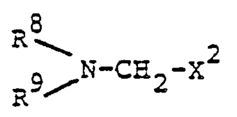

(II)

in der

$X^2$ einen der Reste

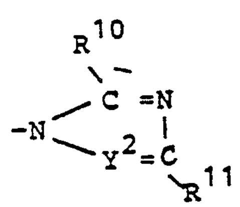

oder

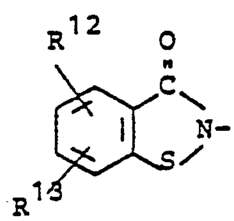

bedeutet,

in denen $Y^2$ für N oder die Gruppe

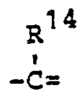

steht und

$R^{10}$ bis $R^{14}$ Wasserstoff, Fluor, Chlor, Niederalkyl ($C_1$-$C_{18}$) oder Niederalkoxy ($C_1$-$C_{18}$) bedeuten,

$R^8$ Alkyl ($C_1$-$C_{18}$), Alkenyl ($C_2$-$C_{18}$), Cycloalkyl ($C_5$-$C_7$), Aralkyl ($C_7$-$C_{12}$), Aminoalkyl ($C_1$-$C_{18}$), Alkylenaminoalkylen, wobei 2 bis 100 Niederalkylengruppen ($C_2$-$C_{12}$) über Aminogruppen der Formel

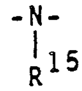

in denen

$R^{15}$ Wasserstoff oder Niederalkyl ($C_1$-$C_{18}$) bedeutet, miteinander verbunden sind, Aryl ($C_6$-$C_{12}$), Alkylaryl ($C_7$-$C_{12}$), Halogenaryl ($C_6$-$C_{12}$), Alkoxy ($C_1$-$C_{18}$), Arylalkoxy ($C_7$-$C_{12}$) oder die Gruppe

$$—CH_2X^2$$

in der $X^2$ die obengenannte Bedeutung hat, bedeutet, $R^9$ Wasserstoff oder $R^8$ bedeutet, wobei $R^8$ die obengenannte Bedeutung hat,
und wobei die Reste $R^8$ und $R^9$ zu einem heterocyclischen 5- oder 6-gliedrigen Ring mit einem oder zwei Stickstoff-, Sauerstoff- und/oder Schwefelatome als Heteroatome verknüpft sein können, bevorzugt.

Beispielsweise seien die folgenden Azolyl-methylamine genannt : 1,2,4-Triazol-1-yl-methyl-di-n-octylamin, Bis-1,2,4-triazol-1-yl-methyl-n-dodecylamin, 2-(1-Morpholinomethyl)-benzisothiazolin-3-on, Imidazol-1-yl-methyl-di-n-octylamin, Bis-imidazol-1-yl-methyl-n-dodecylamin.

Es wurde außerdem ein Verfahren zur Herstellung der Azolyl-methylamine gefunden, das dadurch gekennzeichnet ist, daß man Azole der Formel

$$HX^1 \quad (III)$$

in der $X^1$ einen der Reste

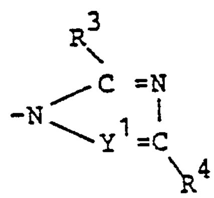

oder

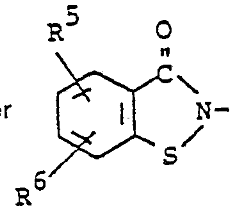

in denen $Y^1$ für Stickstoff oder die Gruppe

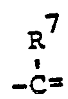

steht und

$R^3$ bis $R^7$ Wasserstoff, Halogen, Nitro, Alkyl ($C_1$-$C_{24}$), Cycloalkyl ($C_3$-$C_{12}$), Alkoxy ($C_1$-$C_{24}$), Halogenalkyl ($C_1$-$C_{24}$), Cyano oder Aralkyl ($C_7$-$C_{24}$) bedeuten,
mit einem Amin der Formel

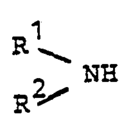

(IV)

in der $R^1$ Alkyl ($C_1$-$C_{24}$), Alkenyl ($C_2$-$C_{24}$), Alkinyl ($C_2$-$C_{24}$), Cycloalkyl ($C_3$-$C_{12}$), Aralkyl ($C_7$-$C_{24}$), Aminoalkyl ($C_1$-$C_{24}$), Alkylenaminoalkylen ($C_2$-$C_{24}$), Aryl ($C_6$-$C_{18}$), Alkylaryl ($C_7$-$C_{24}$), Halogenaryl ($C_6$-$C_{18}$), Alkoxy ($C_1$-$C_{24}$), Arylalkoxy ($C_7$-$C_{24}$) oder die Gruppe

$$—CH_2X^1$$

in der $X^1$ die oben genannte Bedeutung hat, bedeutet,

$R^2$ Wasserstoff oder $R^1$ bedeutet, wobei $R^1$ die oben genannte Bedeutung hat
und wobei die Reste $R^1$ und $R^2$ zu einem heterocyclischen 5- oder 6-gliedrigen Ring mit Stickstoff,

Sauerstoff und/oder Schwefel als Heteroatome verknüpft sein können, und Formaldehyd bei erhöhter Temperatur gegebenenfalls in Gegenwart eines Lösungsmittels umsetzt.

Das erfindungsgemäß Verfahren kann beispielsweise anhand der folgenden Gleichung erläutert werden :

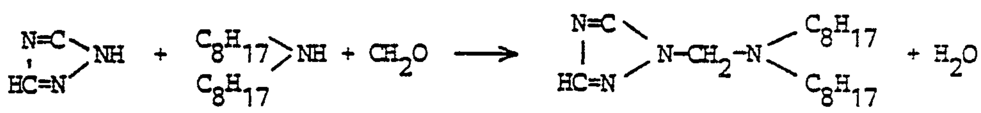

Die Azole für das erfindungsgemäße Verfahren können durch die Formeln

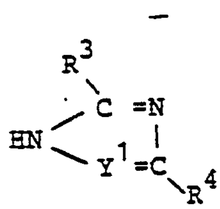 (V) oder 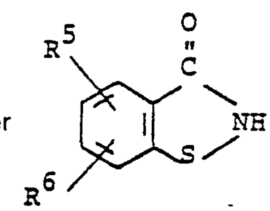 (VI)

in denen $R^3$ bis $R^6$ die oben genannte Bedeutung hat, dargestellt werden. Die Azole für das erfindungsgemäße Verfahren sind an sich bekannt (Angew. Chemie *71*, 753 (1959) ; Chem. Reviews *61*, 87 (1961)).

Beispielsweise seien die folgenden Azole genannt : Pyrazol, Imidazol, 1,2,4-Triazol, Benz-[1,2]-isothiazolin-3-on, 2-Methylimidazol, 2-Methyl-4- und -5-nitroimidazol, Tribromimidazol, Trijodimidazol, Dibromtriazol, Dijodtriazol, Chlor-benzisothiazolinon, Dichlorbenzisothiazolinon, Methyl-benzisothiazolinon, Nitrobenzisothiazolinon.

Amine für das erfindungsgemäße Verfahren sind an sich bekannt und können nach bekannten Verfahren hergestellt werden.

Der Formaldehyd für das erfindungsgemäße Verfahren wird im allgemeinen als Paraformaldehyd oder als Formaldehydlösung in Wasser eingesetzt.

Das erfindungsgemäße Verfahren wird im allgemeinen bei erhöhter Temperatur, bevorzugt im Temperaturbereich von 0 bis 100 °C, insbesondere im Temperaturbereich von 30 bis 40 °C, durchgeführt.

Im allgemeinen wird das erfindungsgemäße Verfahren bei Normaldruck durchgeführt. Es ist jedoch auch möglich, das erfindungsgemäße Verfahren bei einem Unter- oder Überdruck, beispielsweise im Druckbereich von 0,5 bis 10 bar, durchzuführen.

Das erfindungsgemäße Verfahren kann mit oder ohne Lösungsmittel durchgeführt werden. Es werden im allgemeinen Lösungsmittel verwendet, die sich unter den erfindungsgemäßen Reaktionsbedingungen nicht verändern. Als Lösungsmittel seien beispielsweise aprotische Lösungsmittel wie Dichlormethan, Dichlorethan, Trichlorethan, Trichlorethylen, Tetrachlorethylen, Benzol, Toluol, Chlorbenzol, Chlortoluole, Diethylether genannt.

Im allgemeinen führt man das erfindungsgemäße Verfahren wie folgt durch :

Das Amin und das Azol werden mit oder ohne Lösungsmittel vorgelegt und der Formaldehyd zugegeben. Die Reaktion verläuft exotherm unter Wasserabspaltung. Das Reaktionswasser kann durch übliche Methoden entfernt werden, z. B. durch Anlegen von Vakuum, Zugabe von Trockenmitteln oder Azeotropdestillation.

In einer bevorzugten Ausführungsform der erfindungsgemäßen Verfahrens werden das Amin und das Azol in einem inerten Lösungsmittel vorgelegt und die wäßrige Formaldehydlösung zudosiert. Die Temperatur steigt an und wird durch Kühlung im erfindungsgemäßen Temperaturbereich gehalten. Schon vor oder nach Abklingen der Reaktion wird ein Wasserbindemittel zugesetzt und im erfindungsgemäßen Temperaturbereich weitergerührt.

Der Verlauf und der Endpunkt der Umsetzung läßt sich mit Hilfe von spektroskopischen Methoden (NMR oder IR) kontrollieren. Nach Beendigung der Umsetzung wird der Feststoff abfiltriert und die Lösung eingeengt.

Die erfindungsgemäßen Azolyl-methylamine können als Wirkstoffe zur Bekämpfung von Mikroorganismen, im besonderen in technischen Materialien, die durch Mikroorganismen zersetzt werden können, verwendet werden.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor einer mikrobiellen Veränderung oder Zerstörung geschützt werden sollen, Klebstoffe, Leime, Papiere und Kartone, Textilien, Leder, Holz, Anstrichmittel und Kunststoffartikel, Kühlschmiermittel und andere Materialien sein, die durch Mikroorganismen zersetzt werden können. Im Rahmen der zu schützenden Materialien seien auch Teile von Produktionsanlagen, beispielsweise

Kühlwasserkreisläufe, genannt, die durch Mikroorganismen beeinträchtigt werden können. Im Rahmen der vorliegenden Erfindung seien als technische Materialien vorzugsweise Klebstoffe, Leime, Papiere, Kartone, Leder, Holz, Anstrichmittel, Kühlschmiermittel, Kühlkreisläufe genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Substanzen gegen Bakterien, Schimmelpilze, insbesondere holzverfärbende und holzstörende Pilze (Basidiomyceten), sowie gegen Schleimorganismen und Algen.

Es seien beispielsweise Mikroorganismen der folgenden Gattungen genannt :

Alternaria, wie Alternaria tenuis,
Aspergillus, wie Aspergillus niger,
Chaetomium, wie Chaetomium globosum,
Coniophora, wie Coniophora cerebella,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Staphylococcus, wie Staphylococcus aureus,
Pseudomonas, wie Pseudomonas aeruginosa,
Escherichia, wie Escherichia coli,
ferner Grün-, Blau-, Braun- und Kieselalgen.

Je nach ihrem Anwendungsgebiet können die erfindungsgemäßen Wirkstoffe in die üblichen Formulierungen überführt werden, wie Lösungen, Emulsionen, Suspensionen, Pulver, Pasten und Granulate.

Diese können in ansich bekannter Weise hergestellt werden, z. B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei beispielsweise im Falle der Benutzung von Wasserstreckmitteln, gegebenenfalls organische Lösungsmittel wie Alkohole, als Hilfsmittel verwendet werden können.

Organische Lösungsmittel für die Wirkstoffe können beispielsweise Alkohole, wie niedere aliphatische Alkohole, vorzugsweise Ethanol oder Isopropanol, oder Benzylalkohol, Ketone wie Aceton oder Methylethylketon, flüssige Kohlenwasserstoffe, wie Benzinemulsionen, chlorierte Kohlenwasserstoffe, wie 1,2-Dichlorethan, sein.

Die Anwendungskonzentration der erfindungsgemäßen Wirkstoffe richtet sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,01 bis 0,5 Gew.-%, bezogen auf das zu schützende Material.

Die erfindungsgemäßen neuen Wirkstoffe können auch in Mischung mit anderen bekannten Wirkstoffen vorliegen. Beispielsweise seien die folgenden Wirkstoffe genannt : Benzylalkoholmono(poly)hemiformal, Benzimidazolyl-methylcarbamate, Tetramethyl-thiuramdisulfid, Zinksalze von Dialkylthiocarbamaten, 2,4,5,6-Tetrachlorisophthalonitril, Thiazolylbenzimidazol, Mercaptobenzthiazol und Phenolderivate, wie 2-Phenylphenol, (2,2'-Dihydroxy-5,5'-dichlor)-diphenylmethan, 3-Methyl-4-chlor-phenol.

A) Herstellungsbeispiele

Beispiel 1

1,2,4-Triazol-1-yl-methyl-di-n-octylamin

Zu 187 g (0,77 Mol) Di-n-octylamin und 54 g (0,77 Mol) 1,2,4-Triazol in 400 ml Methylenchlorid (oder Ethylenchlorid) wurden 77 g (0,77 Mol) 30 %ige Formaldehydlösung so schnell zugetropft, daß die Temperatur auf 30 bis 35 °C anstieg. Nach Zugabe von 200 g wasserfreiem Natriumsulfat wurde 10 bis 20 Stunden bei 35 bis 40 °C nachgerührt, die Suspension abfiltriert und das Filtrat eingeengt. Ausbeute 244 g (98 %) hellgelbes, klares Öl, $n_D^{20}$ : 1,470 9.

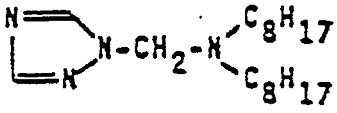

Beispiel 2

Bis-1,2,4-Triazol-1-yl-methyl-n-dodecylamin

Zu einem Gemisch von 37 g (0,2 Mol) n-Dodecylamin, 28 g (0,4 Mol) 1,2,4-Triazol und 300 g wasserfreiem Natriumsulfat in 360 ml Methylenchlorid wurden 40 g (0,4 Mol) 30 %ige wäßrige Formaldehydlösung so schnell zugetropft, daß eine Temperatur von 40 °C erreicht wurde. Dann wurde 5 Stunden bei 40 °C nachgerührt, filtriert und das Filtrat eingeengt. Es wurden 62,5 g (90 %) einer wachsartigen, weißen, unscharf schmelzenden Masse erhalten. Die Struktur wurde durch das NMR-Spektrum bestätigt.

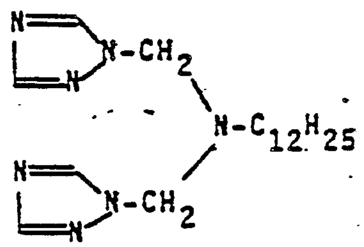

Beispiel 3

2-(1-Morpholinomethyl)-benzisothiazolin-3-on

15,1 g (0,1 Mol) Benzisothiazolin-3-on wurden in 100 ml Methylenchlorid suspendiert. Bei Raumtemperatur wurden 8,7 g (0,1 Mol) Morpholin zugegeben. Dabei ging Benzisothiazolin-3-on in Lösung. 10 g (0,1 Mol) 30 %ige Formalinlösung wurden zugetropft, und anschließend wurde 6 Stunden bei 30 bis 32 °C nachgerührt.

Natriumsulfat wurde zugegeben, bis die Methylenchloridlösung klar war. Nach Filtration wurde das Lösungsmittel am Rotationsverdampfer bei Temperaturen < 35 °C abgezogen. Das zurückbleibende Öl kristallisierte nach kurzem Stehen : 24 g (96 % der Theorie), farblose Kristalle, Fp. 61-63 °C.

| | | | | |
|---|---|---|---|---|
| Elementaranalyse : | ber. C 57,58 | H 5,64 | H 11,19 |
| | gef. C 57,5 | H 5,7 | N 11,1 |

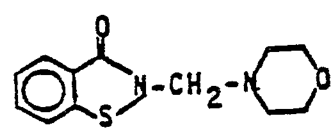

Beispiele 4 bis 82

Entsprechend Beispiel 1 wurden die weiteren Azolylmethylamine hergestellt :

| Beispiel Nr. | Struktur | Ausbeute in % | Brechungsindex ($n_D^{20}$) oder Fp. (°C) |
|---|---|---|---|
| | 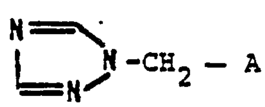 | | |
| | 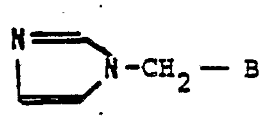 | | |
| | 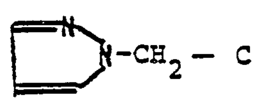 | | |
| | 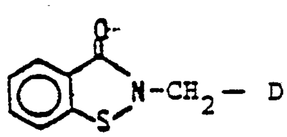 | | |

(Fortsetzung)

| | | | |
|---|---|---|---|
| (4) | 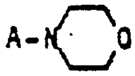 | 80 | 80 - 83°C |
| (5) | 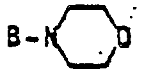 | 100 | 73°C |
| (6) | 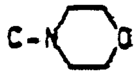 | 100 | 66°C |
| (7) | 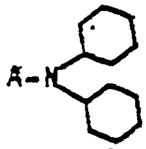 | 96 | 76°C |
| (8) | 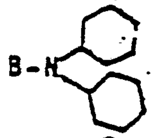 | 93 | 68°C |
| (9) | 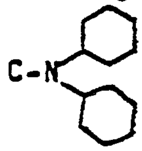 | 85 | 40°C |
| (10) | 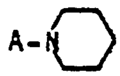 | 98 | 62°C |
| (11) | 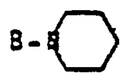 | 96 | 1,5181 |
| (12) | 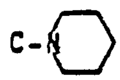 | 100 | 1,5100 |
| (13) | $A-\underset{CH_3}{\underset{\mid}{N}}-(CH_2)_{17}-CH_3$ | 86 | 60°C |
| (14) | $B-\underset{CH_3}{\underset{\mid}{N}}-(CH_2)_{17}-CH_3$ | 88 | 63°C |
| (15) | $C-\underset{CH_3}{\underset{\mid}{N}}-(CH_2)_{17}-CH_3$ | 93 | 38°C |
| (16) | 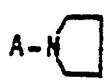 | 96 | 1,5060 |
| (17) | 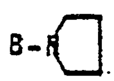 | 96 | 1,5168 |
| (18) | $A-N(CH_3)_2$ | 68 | 1,4800 |
| (19) | $B-N(CH_3)_2$ | 66 | 1,4959 |

(Fortsetzung)

| | | | |
|---|---|---|---|
| 20 | C-$N(CH_3)_2$ | 96 | 1,4832 |
| 21 | A-$N(C_2H_5)_2$ | 95 | 1,4783 |
| 22 | B-$N(C_2H_5)_2$ | 90 | 1,4910 |
| 23 | C-$N(C_2H_5)_2$ | 98 | 1,4791 |
| 24 | 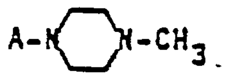 | 89 | 75°C |
| 25 | 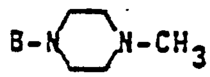 | 92 | 77°C |
| 26 | 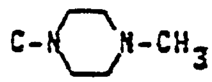 | 95 | 32°C |
| 27 | 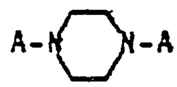 | 70 | 206°C |
| 28 | 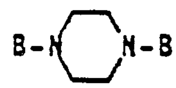 | 31 | 174°C |
| 29 | 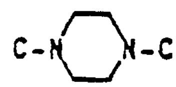 | 100 | 107°C |
| 30 | 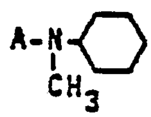 | 100 | 1,5062 |
| 31 | 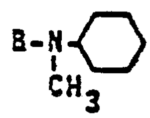 | 100 | 1,5137 |
| 32 | 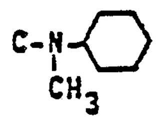 | 95 | 1,5064 |
| 33 | 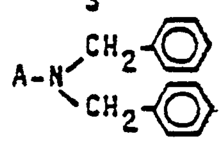 | 94 | 64°C |
| 34 | 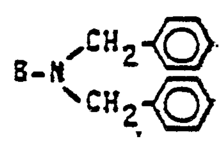 | 100 | 63°C |
| 35 | 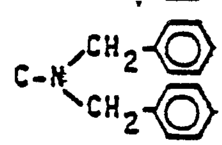 | 95 | 44°C |
| 36 | 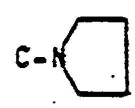 | 99 | 1,5075 |

(Fortsetzung)

| | | | |
|---|---|---|---|
| 37 | 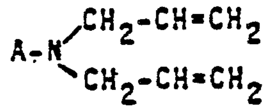 | 97 | 1,4985 |
| 38 | 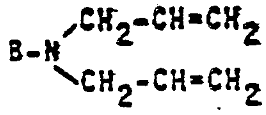 | 91 | 1,5090 |
| 39 | 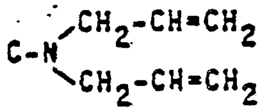 | 98 | 1,4972 |
| 40 | 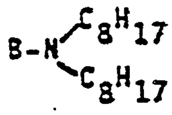 | 81 | 1,4764 |
| 41 | 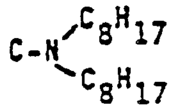 | 73 | 1,4712 |
| 42 | 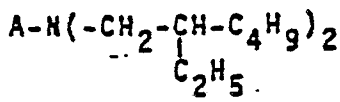 | 95 | 1,4721 |
| 43 | 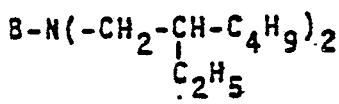 | 83 | 1,4788 |
| 44 | 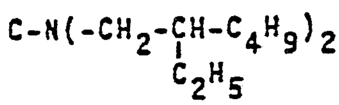 | 97 | 1,4728 |
| 45 | 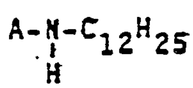 | 86 | 51°C |
| 46 | 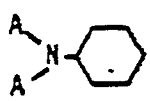 | 46 | hochviskos |
| 47 | 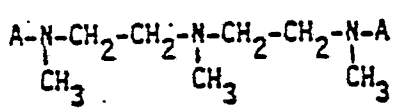 | 78 | 1,5161 |
| 48 | 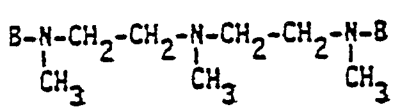 | 70 | 1,5296 |
| 49 | 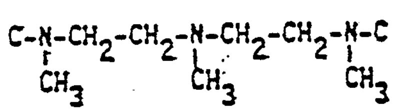 | 85 | 1,5195 |

(Fortsetzung)

| | | | |
|---|---|---|---|
| 50 | 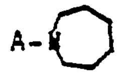 | 98 | 1,5120 |
| 51 | 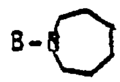 | 95 | 37°C |
| 52 | 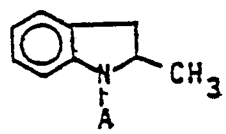 | 85 | - |
| 53 | 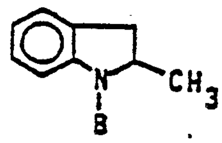 | 88 | - |
| 54 | 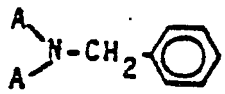 | 97 | 1,5566 |
| 55 | 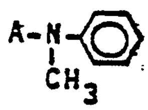 | 94 | 1,5847 |
| 56 | 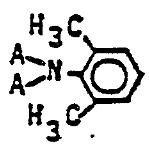 | 77 | 1,5590 |
| 57 | 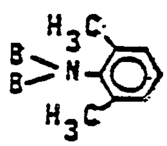 | 100 | 1,5605 |
| 58 | 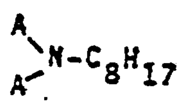 | 93 | 1,4922 |
| 59 | 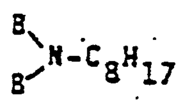 | 100 | 1,5031 |
| 60 | 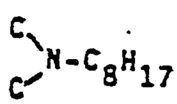 | 100 | 1,4958 |
| 61 | 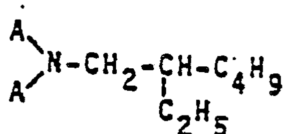 | 98 | 1,4949 |
| 62 | 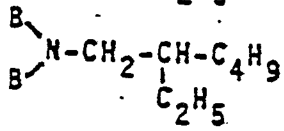 | 100 | 1,5035 |

(Fortsetzung)

| | | | |
|---|---|---|---|
| 63 | 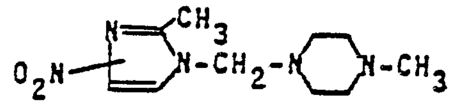 | 95 | 77 °C |
| 64 | 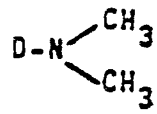 | 91,3 | 83-6 °C |
| 65 | 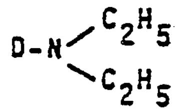 | 89,0 | 41-2 °C |
| 66 | 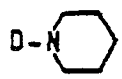 | 86,7 | 88-91 °C |
| 67 | 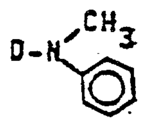 | 92,6 | 96-8 °C |
| 68 | 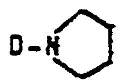 | 81,2 | 79-80 °C |
| 69 | 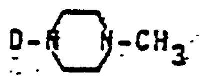 | 91,2 | 102-3 °C |
| 70 | 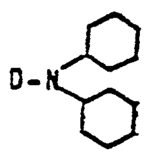 | 81,9 | 93-4 °C |
| 71 | 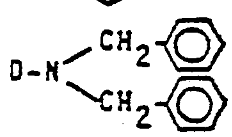 | 83,3 | 62-4 °C |
| 72 | 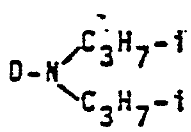 | 83,2 | 62 °C |
| 73 | 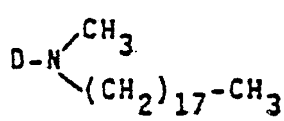 | 82,9 | 63 °C |
| 74 | 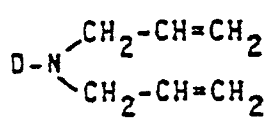 | 84,6 | 1,5961 |
| 75 | 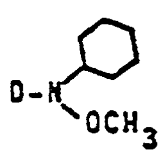 | 77,0 | 89-91°C |

(Fortsetzung)

| | | | |
|---|---|---|---|
| 76 | 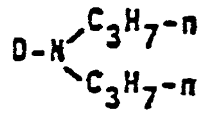 | 89,0 | 1,5712 |
| 77 | 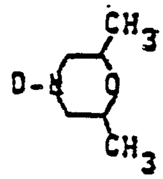 | 93,5 | 1,5738 |
| 78 | 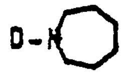 | 87,8 | 64-7°C |
| 79 | 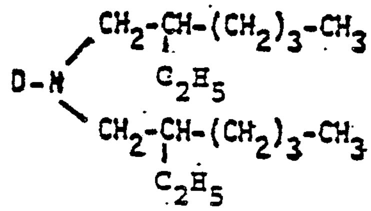 | 80,4 | 1,5288 |
| 80 | 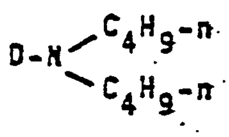 | 87 | 43-5°C |
| 81 | 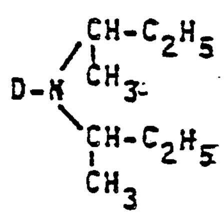 | 94,2 | 46-8°C |
| 82 | 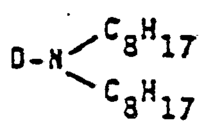 | 93,9 | 1,5288 |
| 87 | 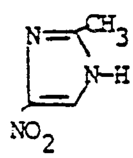 | Vergleichsbeispiel | |

B) Anwendungsbeispiele

Beispiel 83

Zum Nachweis der Wirksamkeit gegen Pilze werden die minimalen Hemm-Konzentrationen (MHK) von erfindungsgemäßen Wirkstoffen bestimmt :

Ein Agar, der aus Bierwürze und Pepton hergestellt wird, wird mit erfindungsgemäßen Wirkstoffen in Konzentrationen von 0,1 mg/l bis 5 000 mg/l versetzt. Nach Erstarren des Agars erfolgt Kontamination mit Reinkulturen der in der Tabelle aufgeführten Testorganismen. Nach 2-wöchiger Lagerung bei 28 °C und 60 bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt, sie ist in der nachstehenden Tabelle angegeben.

Tabelle 1

Angabe der MIK-Werte in mg/l bei der Einwirkung erfindungsgemäßer Substanzen auf Pilze

| Testorganismen | MIK (in mg/l) der Wirkstoffe gemäß den Beispielen: | | | | | | | | | | | | | | | |
|---|---|---|---|---|---|---|---|---|---|---|---|---|---|---|---|---|
| | 1 | 2 | 3 | 40 | 41 | 42 | 43 | 44 | 45 | 59 | 64 | 65 | 66 | 68 | 69 | 70 |
| Alternaria tenuis | 50 | | | 50 | 50 | | | | | | | | 35 | | | 150 |
| Aspergillus niger | 100 | 350 | 150 | 200 | 200 | 500 | 500 | 500 | 500 | 500 | 350 | 150 | 350 | 100 | 200 | 350 |
| Aureobasidium pullulans | 50 | | | 15 | 35 | | | | | | | | 20 | | | 50 |
| Chaetomium globosum | 200 | 200 | 200 | 75 | 75 | 200 | 150 | 200 | 150 | 500 | 150 | 100 | 200 | 200 | 350 | 200 |
| Coniophora cerebella | 20 | | | 10 | 10 | | | | | | | | 75 | | | 20 |
| Lentinus tigrinus | 20 | | | 20 | 20 | | | | | | | | 100 | | | 50 |
| Penicillium glaucum | 50 | 200 | 75 | 50 | 50 | 500 | 200 | 500 | 200 | 500 | 150 | 200 | 150 | 150 | 75 | 150 |
| Polyporus versicolor | 100 | | | 50 | 50 | | | | | | | | 50 | | | 50 |
| Sclerophoma pityophila | 20 | | | 20 | 20 | | | | | | | | 50 | | | 50 |
| Trichoderma viride | 200 | | | 100 | 150 | | | | | | | | 200 | | | 200 |

| Testorganismen | 67 | 71 | 72 | 73 | 74 | 75 | 76 | 77 | 78 | 80 | 82 | 87 |
|---|---|---|---|---|---|---|---|---|---|---|---|---|
| Aspergillus niger | 350 | 200 | 200 | 500 | 200 | 200 | 150 | 200 | 200 | 200 | 100 | > 1000 |
| Chaetomium globosum | 200 | 350 | 200 | 200 | 200 | 100 | 200 | 200 | 350 | 200 | 50 | > 1000 |
| Penicillium glaucum | 350 | 200 | 200 | 200 | 200 | 100 | 100 | 100 | 350 | 100 | 50 | > 1000 |

Beispiel 84

Wirkung gegen Bakterien

Ein Agar, der als Nährmedium Bouillon enthält, wird mit erfindungsgemäßen Wirkstoffen in Konzentrationen von 1 bis 5 000 ppm versetzt. Darauf infiziert man das Nährmedium jeweils mit den in Tabelle II aufgeführten Testorganismen und hält das infizierte Medium 2 Wochen bei 28 °C und 60 bis 70 % rel. Luftfeuchtigkeit. Die MHK ist die niedrigste Konzentration an Wirkstoff, bei der keinerlei Bewuchs durch die verwendete Mikrobenart erfolgt. Die MHK-Werte sind in Tabelle II wiedergegeben.

Tabelle II

Angabe der MHK-Werte in mg/l bei der Einwirkung der unten angegebenen Wirkstoffe auf Bakterien.

| Testorganismen | MHK (in mg/l) der Wirkstoffe gemäß den Beispielen: | | | | | | | | |
|---|---|---|---|---|---|---|---|---|---|
| | 1 | 2 | 3 | 40 | 45 | 64 | 79 | 81 | 87 |
| Escherichia coli | 75 | 50 | 50 | 50 | 50 | < 20 | 50 | 50 | > 1000 |
| Staphylococcus aureus | < 20 | < 20 | 35 | 5 | < 20 | < 20 | < 20 | 50 | > 1000 |
| Pseudomonas aeruginosa | 750 | | | | | | | | |
| Pseudomonas fluorescens | 750 | | | | | | | | |
| Bacillus subtilis | 5 | | | | | | | | |
| Bacterium punctatum | 20 | | | | | | | | |
| Proteus vulgaris | 100 | | | | | | | | |
| Leuconostoc mesenteroides | 20 | | | | | | | | |

Beispiel 85 (Wirkung gegen Schleimorganismen)

Erfindungsgemäße Substanzen werden in Konzentrationen von jeweils 0,1 bis 100 mg/l in Allens Nährlösung (Arch. Microbiol. *17,* 34 bis 53 (1952)), die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1 % Caprolactam enthält, in wenig Aceton gelöst, zur Anwendung gebracht. Kurz vorher wird die Nährlösung mit Schleimorganismen (ca. $10^6$ Keime/ml), die aus bei der Polyamid-Herstellung verwendeten Spinnwasser-Kreisläufen isoliert wurden, infiziert. Nährlösungen, die die minimale Hemmkonzentration (MHK) oder größere Wirkstoffkonzentrationen aufweisen, sind nach 3-wöchiger Kultur bei Raumtemperatur noch völlig klar, d. h. die in wirkstoffreien Nährlösungen nach 3 bis 4 Tagen bemerkbare starke Vermehrung der Mikroben und Schleimbildung unterbleibt.

Tabelle III

MHK-Werte in mg/l bei der Einwirkung der unten angegebenen Substanzen auf Schleimorganismen

| Wirkstoff gemäß Beispiel | MHK in mg/l |
|---|---|
| 1 | 75 |
| 2 | 20 |
| 3 | 5 |
| 40 | 30 |
| 45 | 20 |
| 66 | 3 |
| 68 | 5 |
| 70 | 5 |
| 82 | 3 |

Beispiel 86

Eine Mischkultur von Grün-, Blau-, Braun- und Kieselalgen (Stichococcus bacillaris Naegeli, Euglena gracilis Klebs, Chlorella pyrenoidose Chick, Phormidium foveolarum Gomont, Oscillatoria geminata Meneghini und Phaedodactylum tricornutum Bohlin) wird unter Durchperlen von Luft in Allens Nährlösung (Arch. Microbiol. *17,* 34 bis 53 (1952)), die auf 4 l steriles Wasser 0,2 g Ammoniumchlorid, 4,0 g Natriumnitrat, 1,0 g Dikaliumhydrogenphosphat, 0,2 g Calciumchlorid, 2,05 g Magnesiumsulfat und 0,02 g Eisenchlorid enthält, gegeben. Nach 2 Wochen ist die Nährlösung durch intensives Algenwachstum tief grün-blau gefärbt. Das Absterben der Algen nach Zugabe erfindungsgemäßer Wirkstoffe erkennt man an dem Entfärben der Nährlösung.

Tabelle IV

Algen-abtötende Konzentrationen (mg/l) der unten angegebenen Substanzen

| Wirkstoff gemäß Beispiel | abtötende Konzentration in mg/l |
|---|---|
| 1 | 20 |
| 2 | 75 |
| 3 | 100 |
| 40 | 20 |
| 41 | 15 |
| 45 | 75 |
| 68 | 100 |
| 70 | 100 |
| 82 | 20 |

**Patentansprüche**

1. Azolyl-methylamine der Formel

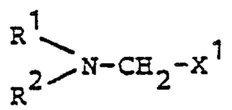

in der $X^1$ einen der Reste

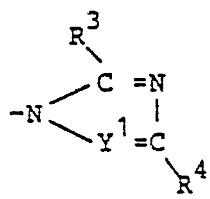

oder

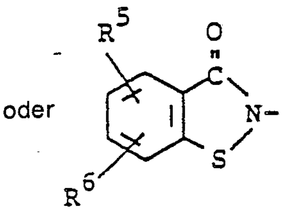

bedeutet, in denen

$Y^1$ für N oder die Gruppe

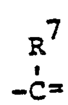

steht und

$R^3$ bis $R^7$ Wasserstoff, Halogen, Nitro, Alkyl ($C_1$-$C_{24}$), Cycloalkyl ($C_3$-$C_{12}$), Alkoxy ($C_1$-$C_{24}$), Cyano oder Aralkyl ($C_7$-$C_{24}$) bedeuten,

$R^1$ Alkyl ($C_1$-$C_{24}$), Alkenyl ($C_2$-$C_{24}$), Alkinyl ($C_2$-$C_{24}$), Cycloalkyl ($C_3$-$C_{12}$), Aralkyl ($C_7$-$C_{24}$), Aminoalkyl ($C_1$-$C_{24}$), Alkylenaminoalkylen ($C_2$-$C_{24}$), Aryl ($C_6$-$C_{18}$), Alkylaryl ($C_7$-$C_{24}$), Halogenaryl ($C_6$-$C_{18}$), Alkoxy ($C_1$-$C_{24}$), Arylalkoxy ($C_7$-$C_{24}$) oder die Gruppe

$$—CH_2X^1$$

in der

$X^1$ die obengenannte Bedeutung hat, bedeutet,

$R^2$ Wasserstoff oder $R^1$ bedeutet,

wobei $R^1$ die obengenannte Bedeutung hat,

und wobei die Reste $R^1$ und $R^2$ in einem heterocyclischen 5- oder 6-gliedrigen Ring mit Stickstoff, Sauerstoff und/oder Schwefel als Heteroatom verknüpft sein können.

2. Azolyl-methylamine nach Anspruch 1 der Formel

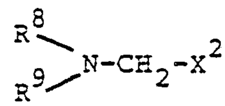

in der $X^2$ einen der Reste

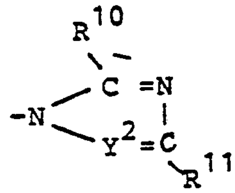

oder

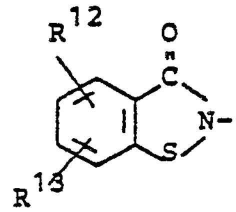

bedeutet, in denen

$Y^2$ für N oder die Gruppe

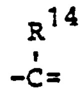

steht und

$R^{10}$ bis $R^{14}$ Wasserstoff, Fluor, Chlor, Niederalkyl ($C_1$-$C_{18}$) oder Niederalkoxy ($C_1$-$C_{18}$) bedeuten,

$R^8$ Alkyl ($C_1$-$C_{18}$), Alkenyl ($C_2$-$C_{18}$), Cycloalkyl ($C_5$-$C_7$), Aralkyl ($C_7$-$C_{12}$), Aminoalkyl ($C_1$-$C_{18}$), Alkylenaminoalkylen, wobei 2 bis 100 Niederalkylengruppen ($C_2$-$C_{12}$) über Aminogruppen der Formel

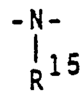

in denen

$R^{15}$ Wasserstoff oder Niederalkyl ($C_1$-$C_{18}$) bedeutet, miteinander verbunden sind, Aryl ($C_6$-$C_{12}$), Alkylaryl ($C_7$-$C_{12}$), Halogenaryl ($C_6$-$C_{12}$), Alkoxy ($C_1$-$C_{18}$), Arylalkoxy ($C_7$-$C_{12}$) oder die Gruppe

$$-CH_2X^2$$

in der $X^2$ die obengenannte Bedeutung hat, bedeutet,

$R^9$ Wasserstoff oder $R^8$ bedeutet, wobei $R^8$ die obengenannte Bedeutung hat, und wobei die Reste $R^8$ und $R^9$ zu einem heterocyclischen 5- oder 6-gliedrigen Ring mit einem oder zwei Stickstoff-, Sauerstoff- und/oder Schwefelatomen als Heteroatome verknüpft sein können.

3. Azoyl-methylamine der Formel

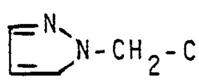

in der

C für 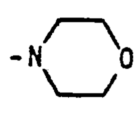 , 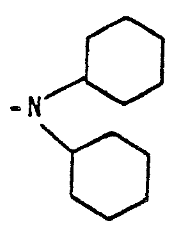 , 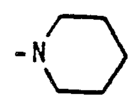 ,

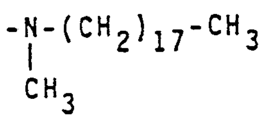 , $-N(CH_3)_2$ , $-N(C_2H_5)_2$ ,

(Fortsetzung)

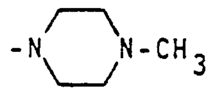 , 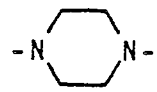 , 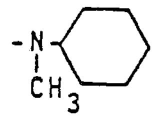 ,

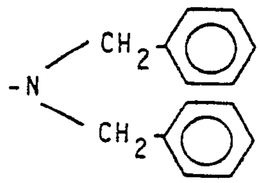 , 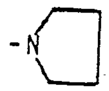 , 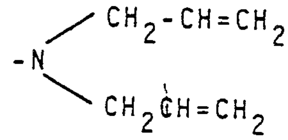 ,

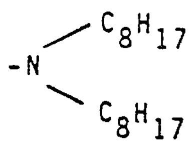 , 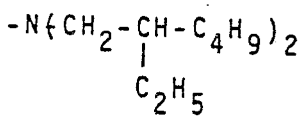 ,

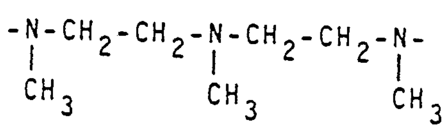 und 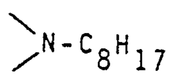 steht,

4. Azoyl-methylamine der Formel

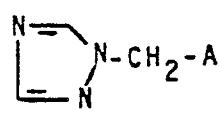 ,

in der

A für 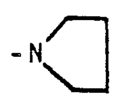 , 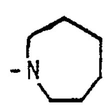 und 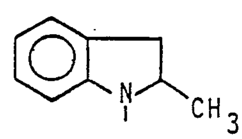 steht.

5. Azoyl-methylamine der Formel

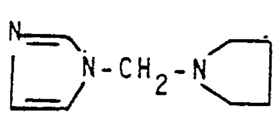 und 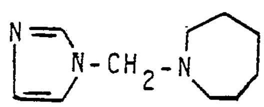

6. Azoyl-methylamine der Formel

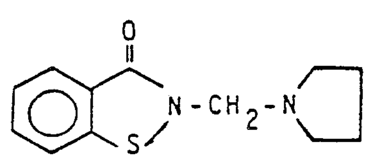 und 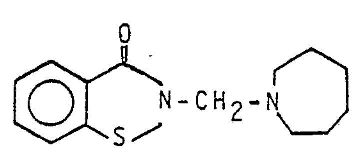

7. Verfahren zur Herstellung von Azolyl-methylaminen gemäß dem Anspruch 1, dadurch gekennzeichnet, daß man ein Azol der Formel

$$HX^1$$

in der $X^1$ einen der Reste

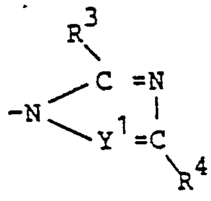

oder

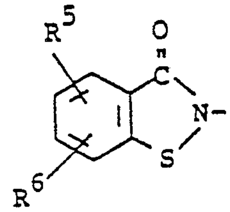

bedeutet, in denen $Y^1$ und $R^3$ bis $R^7$ die in Anspruch 1 angegebene Bedeutung besitzen, mit einem Amin der Formel

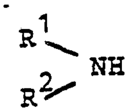

in der $R^1$ und $R^2$ die in Anspruch 1 genannte Bedeutung besitzen, mit Formaldehyd bei erhöhter Temperatur gegebenenfalls in Gegenwart eines Lösungsmittels umsetzt.

8. Verfahren nach Anspruch 7, dadurch gekennzeichnet, daß es im Temperaturbereich von 0 bis 100 °C durchgeführt wird.

9. Mikrobizides Mittel, enthaltend Azolyl-methylamine nach Anspruch 1.

10. Verwendung von Azolyl-methylaminen nach Anspruch 1 zum Schutz technischer Materialien vor mikrobieller Zersetzung.

11. Verwendung nach Anspruch 10, dadurch gekennzeichnet, daß die Azolyl-methylamine in einer Konzentration von 0,001 bis 5 Gew.-%, bezogen auf das zu schützende Material, eingesetzt werden.

## Claims

1. Azolylmethylamines of the formula

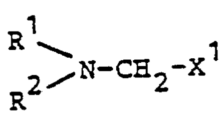

in which $X^1$ denotes one of the radicals

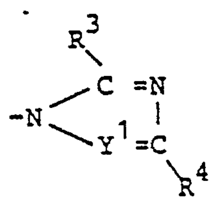

or

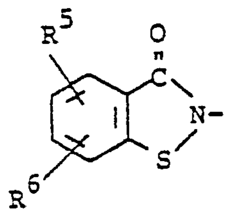

in which $Y^1$ represents N or the group

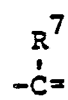

and

$R^3$ to $R^7$ denote hydrogen, halogen, nitro, alkyl ($C_1$-$C_{24}$), cycloalkyl ($C_3$-$C_{12}$), alkoxy ($C_1$-$C_{24}$), cyano or aralkyl ($C_7$-$C_{24}$),

$R^1$ denotes alkyl ($C_1$-$C_{24}$), alkenyl ($C_2$-$C_{24}$), alkinyl ($C_2$-$C_{24}$), cycloalkyl ($C_3$-$C_{12}$), aralkyl ($C_7$-$C_{24}$), aminoalkyl ($C_1$-$C_{24}$), alkyleneaminoalkylene ($C_2$-$C_{24}$), aryl ($C_6$-$C_{18}$), alkylaryl ($C_7$-$C_{24}$), halogenoaryl ($C_6$-$C_{18}$), alkoxy ($C_1$-$C_{24}$), arylalkoxy ($C_7$-$C_{24}$) or the group

$$—CH_2X^1$$

in which $X^1$ has the abovementioned meaning,

$R^2$ denotes hydrogen or $R^1$,

$R^1$ having the abovementioned meaning, and it being possible for the radicals $R^1$ and $R^2$ to be linked

in a heterocyclic 5-membered or 6-membered ring having nitrogen, oxygen and/or sulphur as heteroatoms.

2. Azolylmethylamines according to Claim 1 of the formula

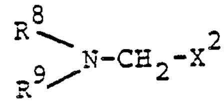

in which $X^2$ denotes one of the radicals

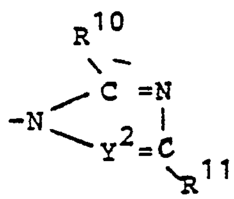

or

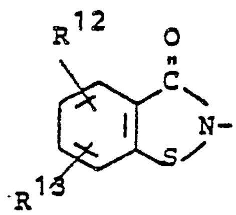

in which $Y^2$ represents N or the group

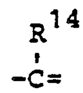

and

$R^{10}$ to $R^{14}$ denote hydrogen, fluorine, chlorine, lower alkyl ($C_1$-$C_{18}$) or lower alkoxy ($C_1$-$C_{18}$),

$R^8$ denotes alkyl ($C_1$-$C_{18}$), alkenyl ($C_2$-$C_{18}$), cycloalkyl ($C_5$-$C_7$), aralkyl ($C_7$-$C_{12}$), aminoalkyl ($C_1$-$C_{18}$), alkyleneaminoalkylene, 2 to 100 lower alkylene groups ($C_2$-$C_{12}$) being bonded together via amino groups of the formula

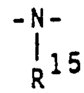

in which $R^{15}$ denotes hydrogen or lower alkyl ($C_1$-$C_{18}$), or aryl ($C_6$-$C_{12}$), alkylaryl ($C_7$-$C_{12}$), halogenoaryl ($C_6$-$C_{12}$), alkoxy ($C_1$-$C_{18}$), arylalkoxy ($C_7$-$C_{12}$) or the group

$$—CH_2X^2$$

in which $X^2$ has the abovementioned meaning,

$R^9$ denotes hydrogen or $R^8$, $R^8$ having the abovementioned meaning, and it being possible for the radicals $R^8$ and $R^9$ to be linked to form a heterocyclic 5-membered or 6-membered ring having one or two nitrogen, oxygen and/or sulphur atoms as heteroatoms.

3. Azoylmethylamines of the formula

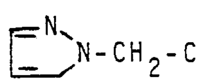

in which C represents

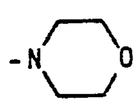 ,

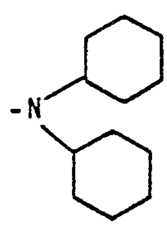 ,

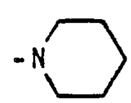 ,

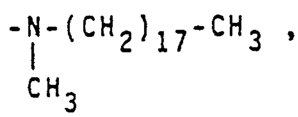 , $-N(CH_3)_2$ , $-N(C_2H_5)_2$ ,

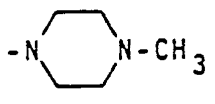 , 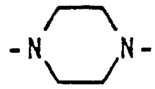 , 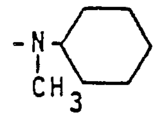 ,

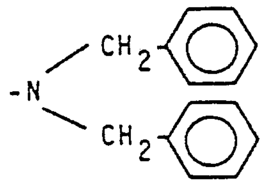 , 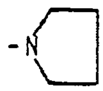 , 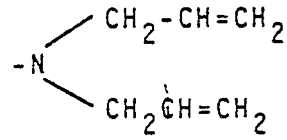 ,

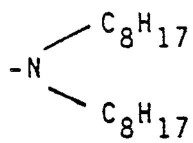 , 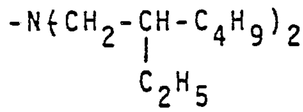 ,

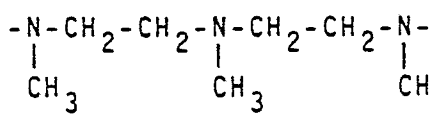 and 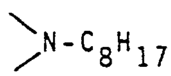

4. Azoylmethylamines of the formula

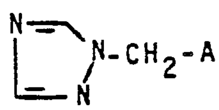 ,

in which A represents 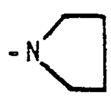 , 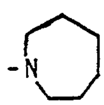 and 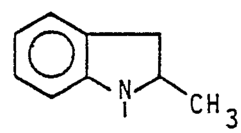 .

5. Azoylmethylamines of the formula

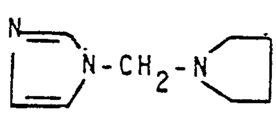 and 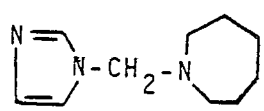

6. Azoylmethylamines of the formula

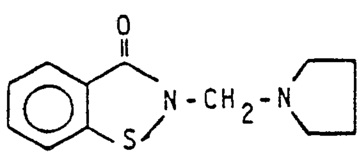 and 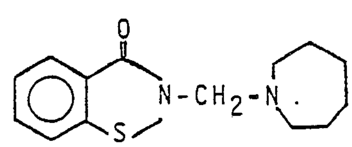

7. Process for the preparation of azolylmethylamines according to Claim 1, characterised in that an azole of the formula

$$HX^1$$

in which $X^1$ denotes one of the radicals

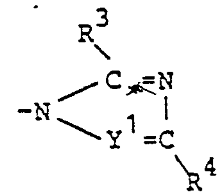

or

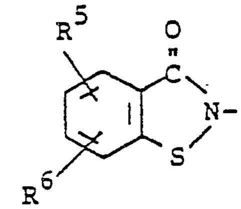

in which $Y^1$ and $R^3$ to $R^7$ have the meaning given in Claim 1, and an amine of the formula

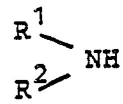

in which $R^1$ and $R^2$ have the meaning given in Claim 1, are reacted with formaldehyde at an elevated temperature optionally in the presence of a solvent.

8. Process according to Claim 7, characterised in that it is carried out in the temperature range from 0 to 100 °C.

9. Microbicidal agent containing azolylmethylamines according to Claim 1.

10. Use of azolylmethylamines according to Claim 1 to protect industrial materials from microbial damage.

11. Use according to Claim 10, characterised in that the azolylmethylamines are employed in a concentration from 0.001 to 5 % by weight relative to the material to be protected.

**Revendications**

1. Azolyl-méthylamines de formule

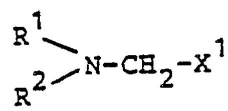

dans laquelle $X^1$ représente l'un des restes

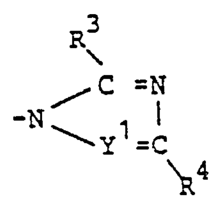

ou

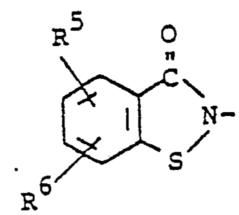

dans lesquels

$Y^1$ représente N ou le groupe

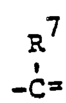

et

$R^3$ à $R^7$ représentent l'hydrogène, un halogène, un groupe nitro, alkyle ($C_1$ à $C_{24}$), cycloalkyle ($C_3$ à $C_{12}$), alkoxy ($C_1$ à $C_{24}$), cyano ou aralkyle ($C_7$ à $C_{24}$),

$R^1$ est un groupe alkyle ($C_1$ à $C_{24}$), alcényle ($C_2$ à $C_{24}$), alcynyle ($C_2$ à $C_{24}$), cycloalkyle ($C_3$ à $C_{12}$), aralkyle ($C_7$ à $C_{24}$), aminoalkyle ($C_1$ à $C_{24}$), alkylène-aminoalkylène ($C_2$ à $C_{24}$), aryle ($C_6$ à $C_{18}$), alkylaryle ($C_7$ à $C_{24}$), halogénaryle ($C_6$ à $C_{18}$), alkoxy ($C_1$ à $C_{24}$), aryloxy ($C_7$ à $C_{24}$) ou le groupe

$$-CH_2X^1$$

dans lequel

$X^1$ a la définition indiquée ci-dessus,

$R^2$ représente l'hydrogène ou $R^1$,

$R^1$ ayant la définition indiquée ci-dessus, et les restes $R^1$ et $R^2$ peuvent être reliés en un noyau

hétérocyclique pentagonal ou hexagonal avec de l'azote, de l'oxygène et/ou du soufre comme hétéroatome.

2. Azolyl-méthylamines suivant la revendication 1, de formule

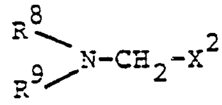

dans laquelle $X^2$ désigne l'un des restes

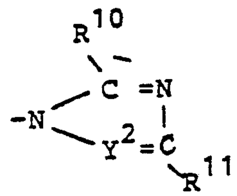

ou

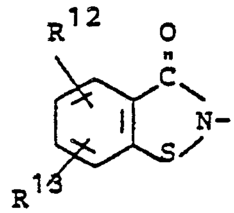

où

$Y^2$ représente N ou le groupe

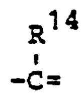

et

$R^{10}$ à $R^{14}$ représentent l'hydrogène, le fluor, le chlore, un groupe alkyle inférieur ($C_1$ à $C_{18}$) ou alkoxy inférieur ($C_1$ à $C_{18}$),

$R^8$ est un groupe alkyle ($C_1$ à $C_{18}$), alcényle ($C_2$ à $C_{18}$), cycloalkyle ($C_5$ à $C_7$), aralkyle ($C_7$ à $C_{12}$), aminoalkyle ($C_1$ à $C_{18}$), alkylèneaminoalkylène, 2 à 100 groupes alkylène inférieurs ($C_2$ à $C_{12}$) étant reliés ensemble par l'intermédiaire de groupes amino de formule

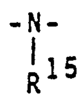

où $R^{15}$ désigne l'hydrogène ou un groupe alkyle inférieur ($C_1$ à $C_{18}$), un groupe aryle ($C_6$ à $C_{12}$), alkylaryle ($C_7$ à $C_{12}$), halogénaryle ($C_6$ à $C_{12}$), alkoxy ($C_1$ à $C_{18}$), arylalkoxy ($C_7$ à $C_{12}$) ou le groupe

$$—CH_2X^2$$

dans lequel $X^2$ a la définition indiquée ci-dessus,

$R^9$ désigne l'hydrogène ou $R^8$, $R^8$ ayant la définition indiquée ci-dessus, et les restes $R^8$ et $R^9$ peuvent être reliés en un noyau hétérocyclique pentagonal ou hexagonal avec un ou deux atomes d'azote, d'oxygène et/ou de soufre comme hétéroatomes.

3. Azolyl-méthylamines de formule

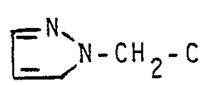

dans laquelle C représente

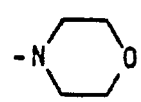 , 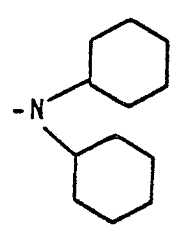 , 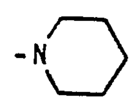 ,

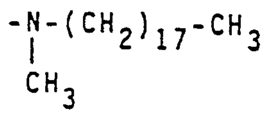 , $-N(CH_3)_2$ , $-N(C_2H_5)_2$ ;

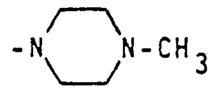 , 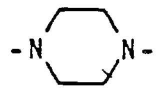 , 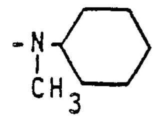 ,

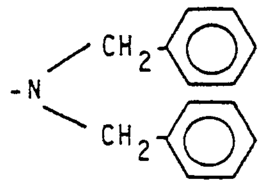 , 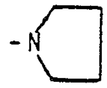 , 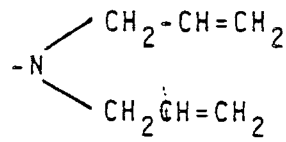 ,

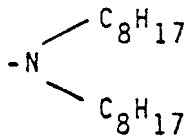 , 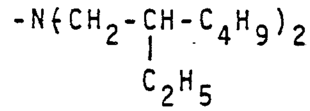 ,

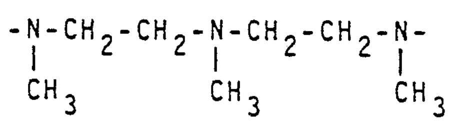 et 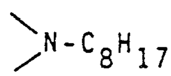

4. Azolyl-méthylamines de formule

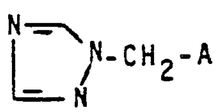

dans laquelle A représente

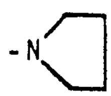 , 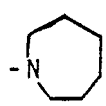 et 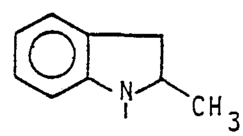

5. Azolyl-méthylamines de formule

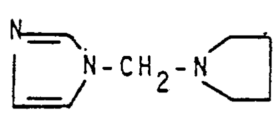 et 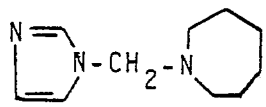

6. Azolyl-méthylamines de formule

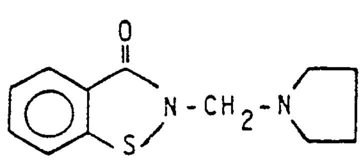 et 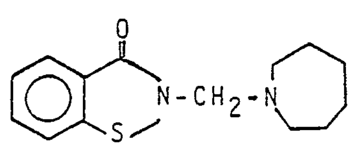

7. Procédé de production d'azolyl-méthylamines suivant la revendication 1, caractérisé en ce qu'on fait réagir un azole de formule

$$HX^1$$

dans laquelle $X^1$ est l'un des restes

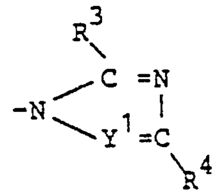

ou

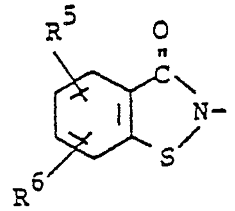

où $Y^1$ et $R^3$ à $R^7$ ont la définition indiquée dans la revendication 1, avec une amine de formule

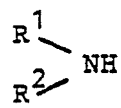

dans laquelle $R^1$ et $R^2$ ont la définition indiquée dans la revendication 1, avec le formaldéhyde à une température élevée, le cas échéant en présence d'un solvant.

8. Procédé suivant la revendication 7, caractérisé en ce qu'il est mis en œuvre dans l'intervalle de température de 0 à 100 °C.

9. Composition microbicide, contenant des azolyl-méthylamines suivant la revendication 1.

10. Utilisation d'azolyl-méthylamines suivant la revendication 1 pour protéger des matériaux industriels d'une décomposition microbienne.

11. Utilisation suivant la revendication 10, caractérisée en ce que les azolyl-méthylamines sont utilisées à une concentration de 0,001 à 5 % en poids par rapport à la matière à protéger.